# EUROPEAN PATENT SPECIFICATION

(11) **EP 2 502 561 B1**
(45) Date of publication and mention of the grant of the patent: **24.12.2014**
(21) Application number: 11305337.5
(22) Date of filing: 25.03.2011
(51) Int. Cl.: A61B 6/00

(54) **Arc-shaped medical imaging equipment**
Bogenförmige medizinische Abbildungsausrüstung
Équipement d'imagerie médicale en forme d'arc

(43) Date of publication of application: 26.09.2012
(73) Proprietor: General Electric Company, Schenectady, NY 12345 (US)
(72) Inventor: Martinez Ferreira, Carlos, 78533 Buc (FR)
(74) Representative: Regimbeau

(56) References cited:
- EP-A1- 1 493 388
- US-A- 4 987 585
- US-A1- 2004 066 880

## Description

### FIELD OF THE INVENTION

The invention relates to the field of medical imaging and more particularly to C-arm equipments.

### BACKGROUND OF THE INVENTION

C-arm equipments are commonly implemented for purposes such as surgical planning, co-registration, image fusion, navigation, implant fitting, surgical validation, etc. During these procedures, it is frequently desirable to observe the patient from several different orientations and is often preferable to do so without the need to reposition the patient. C-arm systems have been developed to meet these needs and are now well known in the medical and surgical arts.

An x-ray source and an x-ray detector are generally mounted on opposing ends of the C-arm gantry such that x-rays emitted by the source are incident on and detectable by the x-ray detector. The source and the detector are positioned such that when an object (e.g., a human extremity) is interposed therebetween and is irradiated with x-rays, the detector produces data representative of characteristics of the interposed object. The data produced is frequently displayed on a monitor or electronically stored.

The C-arm is slidably mounted on a support assembly and defines an axis of rotation (perpendicular to the plane of the C-arm) about which the source and detector are rotatable. By positioning this axis of rotation at or near an object, and by rotating the source and detector around the object in an orbital motion in the plane of the C arm, images of the object taken at a plurality of different orientations can be obtained. These images can be combined to generate a comprehensive three-dimensional image of the object. The process of combining images to produce a comprehensive three-dimensional image is commonly performed with reconstructive software.

The C arm is also typically rotatable on the support assembly, around a longitudinal axis parallel to the plane of the C-arm. By being rotatable about at least two different axes, the C-arm may be positioned at many different orientations about a patient in order to take images from different desirable perspectives. Thus, the mobile C-arm imaging machine greatly increases the efficiency and ease of taking images of a patient before and during a medical procedure.

C-arm imaging machines, such as those commercialised by GE Medical Systems (Innova®), can comprise a C-arm with a rotational axis offset relatively to the plane of the C-arm. The source and the detector are adjacent to the distal ends of the C-arm and are also offset from the plane of the C-arm, so that the axis of rotation of the orbital motion, as well as the longitudinal rotational axis of the C-arm and the image axis intersect on a common point. An example of such a configuration is for example illustrated in US 4,987,585.

Such machines have the advantage of avoiding the complexity of other C-arm machines and of providing a large gap between the source and the detector. However, due to the offset, they present a large gantry volume and the range of movement of the C-arm around the longitudinal offset is limited.

Other C-arm imaging systems have a source and of a detector with an axis extending in the median plane of the C-arm. Although this configuration authorizes smaller gantry volumes and a higher range of movement for the C-arm, the gap between the source and the detector is limited due to the height of the tube extending within the internal space of the C-arm. An example of such a C-arm is described in EP 1,493,388.

Other C-arm equipments also use double slide rails to further increase the capacity of the imaging capacity of the machine. Those equipment are however of high complexity and gantry volume.

US 2004/066880 discloses a radiographic apparatus comprising a source and a detector which faces each other, the source and the detector being rotatable around a sectional axis.

There is therefore a need for a C-arm imaging equipment which at the same time is of a small volume, so as to be easily usable in an operating or exam situation, and presents a large gap between the source and detector so as to allow the device to easily access a patient, while authorising a wide range of movement and positions, without been of a complex structure.

### SUMMARY OF THE INVENTION

It is proposed a medical imaging equipment comprising
- a support assembly,
- an arc-shaped member slidably mounted on the support assembly,
- a radiation source and a detector, said source being mounted on the arc-shaped member in the vicinity of a first distal end of said arc-shaped member and being oriented to radiate along the direction an imaging axis, the detector facing the source along the imaging axis and being mounted on the arc-shaped member, in the vicinity of the second distal end of the arc-shaped member.

The radiation source and the detector are respectively on one side and the other of the mid plane of the arc-shaped member.

The arc-shaped member is mounted rotatable about a rotational axis on the support assembly, said rotational axis extending in said mid plane, the mid plane of the arc shaped member and the imaging axis being angularly offset and crossing at a crossing point between the rotational axis and said imaging axis.

At least part of the source can extend laterally on one side of said arc-shaped member, along the width of said arc-shaped member. The detector can extend laterally on the other side of the arc-shaped member.

Said equipment can also comprise a control unit controlling at the same time a sliding movement of the arc-shaped member and the inclination of said arc-shaped member around the rotational axis so as to maintain the plane defined by the imaging axis and the rotational axis within a selected detection plane.

Also, the arc shaped member can be slidably mounted on a U shaped curved guide mounted on the support assembly and in which the source and / or detector is / are mounted on the arc shaped member with a gap higher than the thickness of the lateral wall of the curved guide, the arc shaped member being slidable in positions in which the source and / or detector is on the side of the curved guide.

Such a C-arm imaging equipment has a gantry of a small volume, offers a large gap between the source and detector as well as a wide range of movement and positions. It is of a very simple mechanical structure.

A medical imaging system can comprise such an equipment as well as calculation means to process the images to generate in particular a comprehensive three-dimensional image.

### BRIEF DESCRIPTION OF THE DRAWINGS

Embodiments of the invention will now be described, by way of example, with reference to the accompanying drawings in which:
Figure 1 schematically illustrates a medical imaging system,
Figure 2 is an perspective view of a C-arm equipment according to an embodiment of the invention,
Figure 3 is a front view of the C-arm equipment of Figure 2,
Figure 4 is a side view of the C-arm equipment of Figure 2, and
Figure 5a and 5b are detail views of the source and detector mountings at the distal ends of the C-arm equipment of Figure 2.

### DETAILED DESCRIPTION OF THE INVENTION

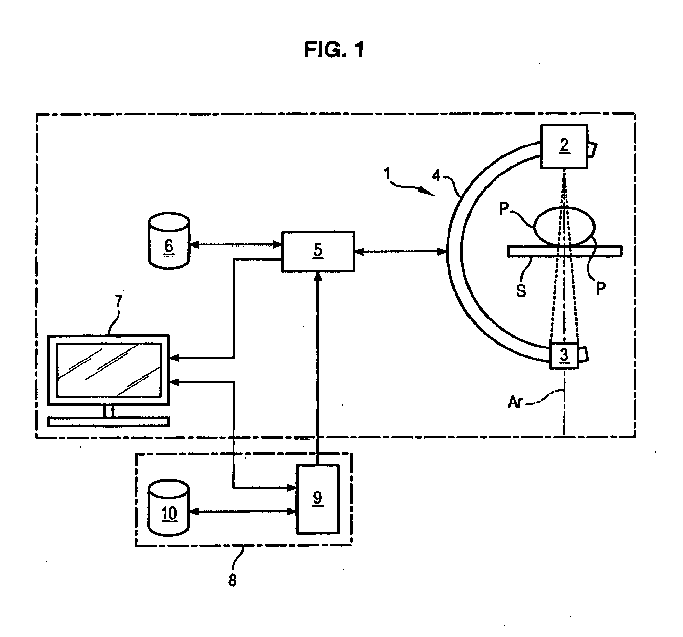

The medical imaging system of figure 1 comprises a C-arm equipment 1 with a source 2 and a detector 3 supported by an arc-shaped member 4 (also commonly known as a C-arm or C-arc). Equipment 1 also comprises a support S for receiving a patient P to be examined, a control unit 5, a storage unit 6 and a display unit 7.

Source 2 and detector 3 are placed opposite at both ends of said arc-shaped-member 4. Source 2 is for example an X-ray source and is to emit a radiation beam B along the direction of an imaging axis Ar through the zone of the patient to be examined. Detector 4 is to detect the radiations emitted by said source 2 emitted through the patient. It can be a CCD sensor or a direct digital detector which directly converts the radiations detector into digital signals.

The control unit 5 is linked to the C-arm equipment 1 and controls acquisition by fixing several parameters such as the dose radiation to be emitted and the positions and movements of the C-arm. The storage unit 6 records the acquired parameters and images. It is connected to the control unit 5 and can be located inside or outside said control unit 5. The display unit 7 can be a display device of any known type. It is connected to the control unit 5 and allows a practitioner to control acquisition of radiological images.

Control unit 5, storage unit 6 and display unit 7 are also coupled to a processing system 8, which comprises a calculation unit 9 and storage unit 10. The processing system 8 receives images acquired and stored in storage unit 6 and processes the images to generate in particular a comprehensive three-dimensional image.

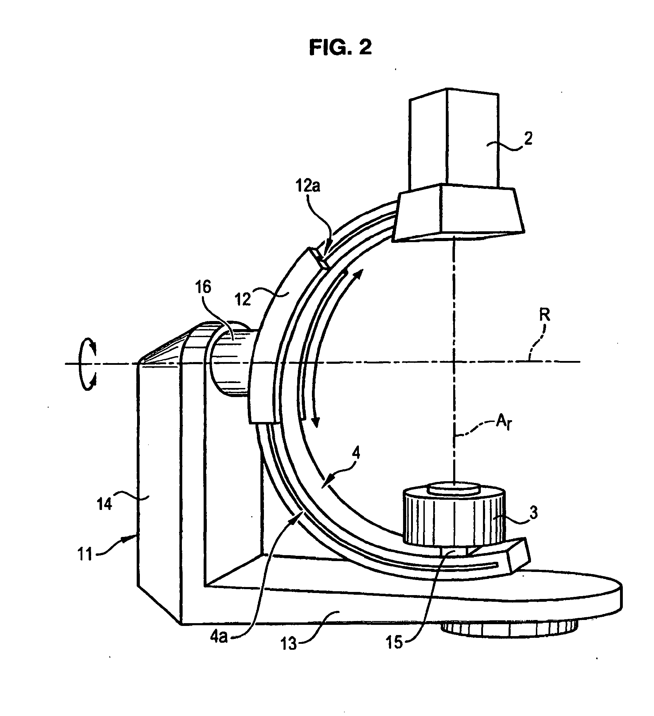

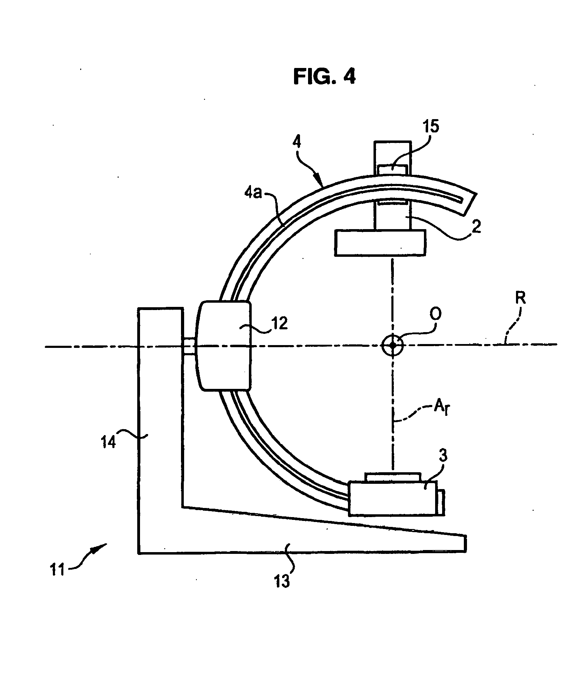

The C-arm equipment 1 more particularly illustrated on figures 2 to 4 comprises a support assembly 11 and a curved guide 12 which is mounted on said support assembly 11. Said curved guide 12 slidably carries C-arm 4, so that said C-arm 4 can slide within said curve guide 12, thereby providing the orbital rotation movement of source 2 and detector 4.

In the case of a C-arm equipment supported on the ground as illustrated in the figures, support assembly 11 can be L-shaped and includes a base 13 and a riser 14. The base 13 is adjacent to the floor, and can comprise wheels in order to be movable with respect to the floor. The riser 14 extends upward and is affixed to the arm base 13. Other forms of support assembly 11 can be contemplated, in particular in cases of C-arms with a support carrier mounted on the ceiling.

Curved guide 12 is for example a U shaped rail. Each of its lateral walls can comprise at least a groove 12a extending along its length, which cooperates with corresponding projections 4a extending along the sides of C-arm 4.

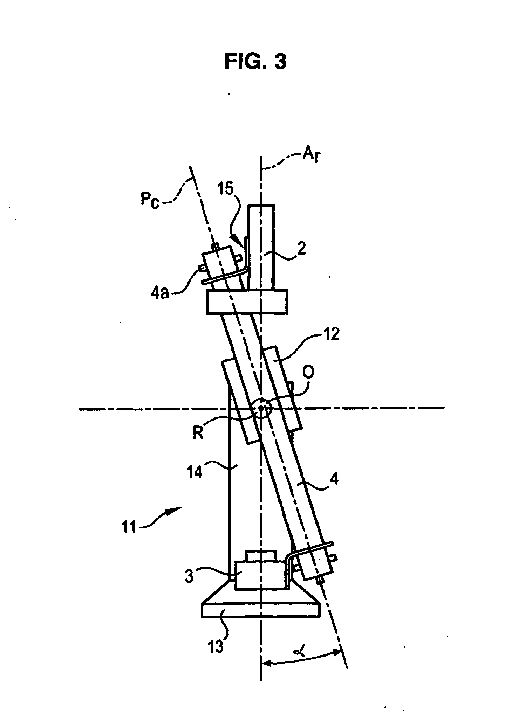

Curved guide 12 is pivotally mounted on riser 14 through a rotating support arm 16 which allows curved guide 12 and C-arm 4 to rotate about a rotational axis R. Said rotational axis R extends in the mid plane Pc (figure 3) of the L shaped support assembly 11. It is parallel to the main direction of base 13 (horizontal) and perpendicular to the riser 14.

Source 2 and detector 3 are respectively on one side and the other of the mid plane Pc of the C-arm 4. The imaging axis Ar defined by source 2 and detector 3 is thus inclined relatively to said mid plane Pc of C-arm 4. Said mid plane Pc and said imaging axis Ar are angularly offset (angle α) and cross at a crossing point O between the rotational axis and said imaging axis. Source 2 being in the vicinity of a first distal end of the C-arm, at least part of it extends laterally on one side of said C-arm 4, along the width of said C-arm 4. Part of it can also extend above. Detector 3 faces said source 2 and extends laterally on the other side of C-arm 4, in the vicinity of the opposite distal end of the C-arm.

Control unit 5 is programmed to command various internal motors (not represented), which the arc-shaped member rotational movement around the R axis as well as the sliding movement of said member 4 within curve guide 12, in order to move source 2 and detector 3 as follows.

C-arm 4 can be inclined by rotation around rotational axis R so as to permit acquisition in any detection plane. Once source 2 and detector 3 positioned so that the plane defined by the imaging axis and the rotational axis coincides with the detection plane that one wants to explore, an orbital movement of said source 2 and detector 3 in such a detection plane can be controlled by said control unit 5. Said orbital movement can be achieved through controlling at the same time a sliding movement of C-arm 4 within curved guide 12 and a corresponding modification of the inclination of C-arm 4 around rotational axis R so as to maintain the plane defined by the imaging axis and the rotational axis within the selected detection plane.

For example, when the detection plane is vertical, the C-arm is angularly inclined relatively to the vertical from angle which is maximum when the imaging axis of source 2 and detector 3 is itself vertical and which decreases when said imaging axis is toggled towards rotational axis R.

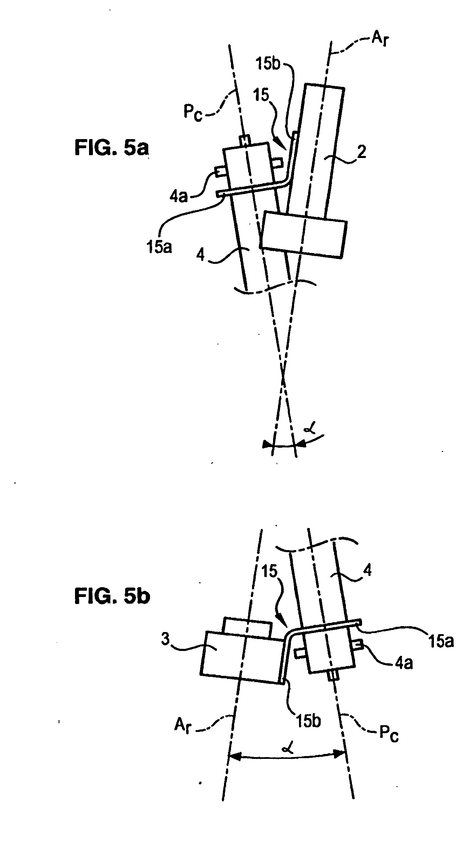

As represented on Figures 5a and 5b, source 2 and detector 3 can be supported on the C arm 4 through mounting plates 15 configured in a L. One branch (branch 15a) is internally fixed to the C-arm 4; the second branch (branch 15b) is externally fixed on source 2 or on detector 3.

The two branches 15a, 15b of such a mounting plate in L are bent relatively to each other so as achieve the angular offset (angle α) between the C-arc mid plane Pc and the imaging axis of source 2 and detector 3.

Further, C-arm 4 is fixed on branch 15a in such a way and C-arm 4 is dimensioned so, that a gap is left between said C-arm 4 and branch 15b bearing source 2 or detector 3. This gap is higher than a thickness of the lateral walls of the curved guide 12 so that the sliding movement of the C-arm along the curved guide 12 is not blocked by mounting plates 15, the source 2 or the detector 3 when said source 2 or detector 3 reach the zone of the curved guide 12. The C-arm can continue to slide with the source 2 and the detector 3 being on the side of the curved guide 12, the C-arm thus sliding with a large range of movement.

## Claims

1. Medical imaging equipment comprising
- a support assembly (11),
- an arc-shaped member (4) slidably mounted on the support assembly (11),
- a radiation source (2) and a detector (3), said source (2) being mounted on the arc-shaped member (4) in the vicinity of a first distal end of said arc-shaped member (4) and being oriented to radiate along the direction an imaging axis (Aᵣ), the detector facing the source (2) along the imaging axis (Aᵣ) and being mounted on the arc-shaped member (4), in the vicinity of the second distal end of the arc-shaped member (4),
**characterized in that**:
- the radiation source (2) and the detector (3) are respectively on one side and the other of the mid plane (Pc) of the arc-shaped member (4),
- the arc-shaped member (4) is mounted rotatable about a rotational axis (R) on the support assembly (11), said rotational axis (R) extending in the mid plane (Pc), and
- the mid plane (Pc) of the arc shaped member (4) and the imaging axis (Ar) are angularly offset and cross at a crossing point between the rotational axis (R) and said imaging axis (Ar).

2. The equipment of claim 1, in which at least part of the source (2) extends laterally on one side of said arc-shaped member (4), along the width of said arc-shaped member (4).

3. The equipment of claim 2, in which the detector (3) extends laterally on the other side of the arc-shaped member (4).

4. The equipment of claim 1, in which the arc-shaped member (4) is mounted rotatable about a rotational axis (R) on the support assembly (11), said equipment also comprising a control unit (5) controlling at the same time a sliding movement of the arc-shaped member (4) and the inclination of said arc-shaped member (4) around said rotational axis (R) so as to maintain the plane defined by the imaging axis (Ar) and the rotational axis (R) within a selected detection plane.

5. The equipment of claim 1 in which the arc shaped member (4) is slidably mounted on a U shaped curved guide (12) mounted on the support assembly (11) and in which the source (2) and / or detector (3) is / are mounted on the arc shaped member (4) with a gap higher than the thickness of the lateral wall of the curved guide (12), the arc shaped member (4) being slidable in positions in which the source (2) and / or detector (3) is on the side of the curved guide (12).

6. The equipment of claim 5 in which the source (2) and / or the detector (3) is supported on the arc shaped member (4) through a mounting plate (15) configured in a L.

7. The equipment of claim 6 in which the two branches (15a, 15b) of such a mounting plate (15) in L are bent relatively to each other with an angle corresponding to the angular offset (α) between the arc shaped member (4) and the imaging axis (Ar).

8. A medical imaging system comprising a medical imaging equipment according to claim 1,
- a radiation source (2) and a detector (3), said source (2) being mounted on the arc-shaped member (4) in the vicinity of a first distal end of said arc-shaped member (4) and being oriented to radiate along the direction an imaging axis (Ar), the detector (3) facing the source (2) along the imaging axis (Ar) and being mounted on the arc-shaped member (4), in the vicinity of the second distal end of the arc-shaped member (4),
said medical imaging system also comprising a processing system (8) comprising calculation means (9) processes the images to generate in particular a comprehensive three-dimensional image,
**characterized in that**:
- the radiation source (2) and the detector (3) are respectively on one side and the other of the mid plane (Pc) of the arc-shaped member (4),
- the arc-shaped member (4) is mounted rotatable about a rotational axis (R) on the support assembly (11), and
- the mid plane (Pc) of the arc shaped member (4) and the imaging axis (Ar) are angularly offset and cross at a crossing point between the rotational axis (R) and said imaging axis (Ar).

## Patentansprüche

1. Ausrüstung zur medizinischen Bildgebung, umfassend
- eine Trägervorrichtung (11),
- ein bogenförmiges Element (4), das auf der Trägervorrichtung (11) verschiebbar montiert ist,
- eine Strahlenquelle (2) und einen Detektor (3), wobei die Quelle (2) auf dem bogenförmigen Element (4) in der Nähe eines ersten distalen Endes des bogenförmigen Elements (4) montiert ist und so orientiert ist, dass sie entlang der Richtung einer Bildgebungsachse (Aᵣ) strahlt, und wobei der Detektor der Quelle (2) entlang der Bildgebungsachse (Aᵣ) gegenübersteht und auf dem bogenförmigen Element (4) in der Nähe des zweiten distalen Endes des bogenförmigen Elements (4) montiert ist,
- wobei das bogenförmige Element (4) auf der Trägervorrichtung (11) um eine Rotationsachse (R) drehbar montiert ist,
**dadurch gekennzeichnet, dass**:
die Rotationsachse (R) sich in der Mittelebene (Pc) erstreckt, und
die Strahlenquelle (2) und der Detektor (3) auf einer Seite bzw. auf der anderen Seite der Mittelebene (Pc) des bogenförmigen Elements (4) fest montiert sind, so dass die Mittelebene (Pc) des bogenförmigen Elements (4) und die Bildgebungsachse (Ar) einen Winkelversatz aufweisen und sich an einem Kreuzungspunkt zwischen der Rotationsachse (R) und der Bildgebungsachse (Ar) kreuzen.

2. Ausrüstung nach Anspruch 1, wobei zumindest ein Teil der Quelle (2) sich in Längsrichtung auf einer Seite des bogenförmigen Elements (4) erstreckt, entlang der Breite des bogenförmigen Elements (4).

3. Ausrüstung nach Anspruch 2, wobei der Detektor (3) sich in Längsrichtung auf der anderen Seite des bogenförmigen Elements (4) erstreckt.

4. Ausrüstung nach Anspruch 1, wobei das bogenförmige Element (4) um eine Rotationsachse (R) drehbar auf der Trägervorrichtung (11) montiert ist, wobei die Ausrüstung ferner eine Steuereinheit (5) umfasst, welche gleichzeitig die Verschiebung des bogenförmigen Elements (4) und die Neigung des bogenförmigen Elements (4) um die Rotationsachse (R) steuert, um dadurch die durch die Bildgebungsachse (Ar) und die Rotationsachse (R) definierte Ebene innerhalb einer ausgewählten Detektionsebene zu halten.

5. Ausrüstung nach Anspruch 1, wobei das bogenförmige Element (4) auf einer U-förmigen gekrümmten Führung (12), die auf der Trägervorrichtung (11) montiert ist, verschiebbar montiert ist, und wobei die Quelle (2) und/oder der Detektor (3) auf dem bogenförmigen Element mit einer Lücke, die größer ist als die Dicke der Seitenwand der gekrümmten Führung (12), montiert ist/sind, wobei das bogenförmige Element (4) in Positionen verschiebbar ist, bei denen die Quelle (2) und/oder der Detektor (3) auf der Seite der gekrümmten Führung (12) ist.

6. Ausrüstung nach Anspruch 5, wobei die Quelle (2) und/oder der Detektor (3) auf dem bogenförmigen Element (4) durch eine Montageplatte (15) in Form eines L getragen wird.

7. Ausrüstung nach Anspruch 6, wobei die zwei Zweige (15a, 15b) einer solchen Montageplatte (15) in L-Form relativ zueinander um einen Winkel gebogen sind, der dem Winkelversatz (α) zwischen dem bogenförmigen Element (4) und der Bildgebungsachse (Ar) entspricht.

8. Medizinisches Bildgebungssystem, umfassend eine Ausrüstung zur medizinischen Bildgebung nach Anspruch 1, wobei das medizinische Bildgebungssystem ferner ein Verarbeitungssystem (8) umfasst, welches Rechenmittel (9) umfasst, die dafür konfiguriert sind, die Bilder zu verarbeiten, um insbesondere ein umfassendes dreidimensionales Bild zu erzeugen.

## Revendications

1. Equipement d'imagerie médicale comprenant
- un ensemble support (11),
- un élément en forme d'arc (4) monté de manière coulissante sur l'ensemble support (11),
- une source de rayonnement (2) et un détecteur (3), ladite source (2) étant montée sur l'élément en forme d'arc (4) au voisinage d'une première extrémité distale dudit élément en forme d'arc (4) et étant orientée pour rayonner le long de la direction d'un axe d'imagerie (Aᵣ), le détecteur faisant face à la source (2) le long de l'axe d'imagerie (Aᵣ) et étant monté sur l'élément en forme d'arc (4), au voisinage de la seconde extrémité distale de l'élément en forme d'arc (4),
- l'élément en forme d'arc (4) étant monté de manière rotative autour d'un axe de rotation (R) sur l'ensemble support (11),
**caractérisé en ce que** :
ledit axe de rotation (R) s'étend dans le plan médian (Pc), et
la source de rayonnement (2) et le détecteur (3) sont respectivement montés de manière fixe de part et d'autre du plan médian (Pc) de l'élément en forme d'arc (4), de telle sorte que
le plan médian (Pc) de l'élément en forme d'arc (4) et l'axe d'imagerie (Ar) soient décalés angulairement et se croisent au niveau d'un point d'intersection entre l'axe de rotation (R) et ledit axe d'imagerie (Ar).

2. Equipement selon la revendication 1, dans lequel au moins une partie de la source (2) s'étend latéralement sur un côté dudit élément en forme d'arc (4), le long de la largeur dudit élément en forme d'arc (4) .

3. Equipement selon la revendication 2, dans lequel le détecteur (3) s'étend latéralement sur l'autre côté de l'élément en forme d'arc (4).

4. Equipement selon la revendication 1, dans lequel l'élément en forme d'arc (4) est monté de manière rotative autour d'un axe de rotation (R) sur l'ensemble support (11), ledit équipement comprenant également une unité de commande (5) commandant en même temps un mouvement coulissant de l'élément en forme d'arc (4) et l'inclinaison dudit élément en forme d'arc (4) autour dudit axe de rotation (R) de sorte à maintenir le plan défini par l'axe d'imagerie (Ar) et l'axe de rotation (R) à l'intérieur d'un plan de détection sélectionné.

5. Equipement selon la revendication 1, dans lequel l'élément en forme d'arc (4) est monté de manière coulissante sur un guide incurvé en U (12) monté sur l'ensemble support (11) et dans lequel la source (2) et/ou le détecteur (3) sont montés sur l'élément en forme d'arc (4) avec un jeu supérieur à l'épaisseur de la paroi latérale du guide incurvé (12), l'élément en forme d'arc (4) étant coulissant dans des positions dans lesquelles la source (2) et/ou le détecteur (3) sont sur le côté du guide incurvé (12).

6. Equipement selon la revendication 5, dans lequel la source (2) et/ou le détecteur (3) sont supportés sur l'élément en forme d'arc (4) par le biais d'une plaque de montage (15) configurée en L.

7. Equipement selon la revendication 6, dans lequel les deux branches (15a, 15b) d'une telle plaque de montage (15) en L sont courbées l'une par rapport à l'autre selon un angle correspondant au décalage angulaire (α) entre l'élément en forme d'arc (4) et l'axe d'imagerie (Ar).

8. Système d'imagerie médicale comprenant un équipement d'imagerie médicale selon la revendication 1,
ledit système d'imagerie médicale comprenant également un système de traitement (8) comprenant des moyens de calcul (9) configuré pour traiter les images pour générer en particulier une image tridimensionnelle détaillée.
